# EUROPÄISCHE PATENTSCHRIFT

(11) **EP 4 433 110 B1**
(45) Veröffentlichungstag und Bekanntmachung des Hinweises auf die Patenterteilung: **05.11.2025**
(21) Anmeldenummer: 22814344.2
(22) Anmeldetag: 08.11.2022
(51) Int. Cl.: A61L 31/12, A61L 31/14, A61F 2/14

(54) **STÜTZMEMBRAN SOWIE VERFAHREN ZUR HERSTELLUNG EINER STÜTZMEMBRAN**
SUPPORTING MEMBRANE AND METHOD FOR PRODUCING A SUPPORTING MEMBRANE
MEMBRANE DE SUPPORT ET PROCÉDÉ DE FABRICATION D'UNE MEMBRANE DE SUPPORT

(30) Priorität: 17.11.2021 DE 102021130009
(43) Veröffentlichungstag der Anmeldung: 25.09.2024
(73) Patentinhaber: Universität Rostock, 18055 Rostock (DE)
(72) Erfinder: BACKHAUS, Franziska, 18057 Rostock (DE); EICKNER, Thomas, 18059 Rostock (DE); GRABOW, Niels, 18055 Rostock (DE); HIMMLER, Marcus, 90425 Nürnberg (DE); ILLNER, Sabine, 18059 Rostock (DE); SCHÜMANN, Kerstin, 18057 Rostock (DE); TROSAN, Peter, 18196 Kessin (DE); FUCHSLUGER, Thomas Armin, 18119 Rostock (DE)
(74) Vertreter: Heinemeyer, Karsten
(86) Internationale Anmeldenummer: PCT/EP2022/081088
(87) Internationale Veröffentlichungsnummer: WO 2023/088729

(56) Entgegenhaltungen:
- WO-A2-2010/083173

## Beschreibung

Die Erfindung betrifft eine Stützmembran für ein Implantat zur Behandlung von Endothelerkrankungen einer Augenhornhaut sowie ein Verfahren zu dessen Herstellung. Die Stützmembran verfügt über ein Substrat, das für eine Befestigung an einer Rückwand der Augenhornhaut eines Menschen oder eines Tieres geeignet ist.

Bei bestimmten Erkrankungen der Augenhornhaut (Cornea), die den transparenten Teil des Auges direkt vor der Pupille und der Regenbogenhaut (Iris) darstellt, wird diese im Wege einer Transplantation zumindest teilweise ersetzt. Etwa 6000 Hornhauttransplantationen werden derzeit jährlich in Deutschland durchgeführt. Diese Operationen sind erforderlich, da Eintrübungen und Verletzungen der Hornhaut das Sehvermögen teilweise stark einschränken oder sogar zur Erblindung führen können. Bei speziellen Fehlfunktionen der Augenhornhaut, die z.B. durch die sogenannte Fuchs-Endotheldystrophie oder die pseudophake bullöse Keratopathie verursacht werden, wird die hiervon betroffene Endothelschicht der Hornhaut, die in-vivo praktisch nicht regenerationsfähig ist, durch ein Transplantat ersetzt.

Das Endothel bildet die sehr dünne und am weitesten innen gelegene Schicht der Hornhaut. Die Endothelzellen sind wichtig, um die Transparenz der Hornhaut aufrechtzuerhalten. Normalerweise diffundiert die Flüssigkeit (Kammerwasser) aus dem inneren des Auges langsam in die mittlere Hornhautschicht (Stroma). Dem Endothel kommt hierbei die Aufgabe zu, diese Flüssigkeit wieder aus dem Stroma herauszupumpen bzw. einen passiven Gradienten aufzubauen. Ohne diese Pumpfunktion würde die Hornhaut aufquellen und schließlich eintrüben.

Zwischen der mittleren Hornhautschicht (Stroma) und der Endothelschicht befindet sich die Descemet-Membran. Diese durchsichtige, homogene Membran besteht aus verschiedenen Schichten und nimmt im Laufe des Lebens an Dicke zu. Durch den hohen Anteil an Kollagenfasern ist die Descemet-Membran eine sehr widerstandsfähige und elastischste Schicht der gesamten Hornhaut und schützt das Endothel vor Infektionen und mechanischen oder chemischen Verletzungen. Aufgrund ihrer Widerstandsfähigkeit sorgt die Descemet-Membran für eine optimale Stabilität der Hornhaut. Bei einer Schädigung besteht wiederum die Gefahr, dass Kammerwasser in die Hornhaut eindringt und zum Aufquellen und damit zur Trübung der Hornhaut führen. Beim Keratokonus beispielsweise wölbt sich die Hornhaut kegelförmig, wobei bei einem fortschreitenden Ausdehnen die Gefahr besteht, dass die Descemet-Membran einreißt. Eine Behandlung erfolgt hierbei üblicherweise entweder konventionell mittels einer perforierenden Hornhauttransplantation, bei der die verformte Hornhaut vollständig oder sehr tiefgreifend herausgeschnitten und durch eine Spenderhornhaut ersetzt wird. Isolierte Hornhautrückwand-Transplantation (DMEK - Descemet Membran Endothelial Keratoplasty) werden durchgeführt bei isolierten Erkrankungen der Hornhautendothelzellen und / oder der Descemetmembran - es handelt sich hierbei um ein vergleichsweise schonendes, minimalinvasives Transplantationsverfahren, bei dem die erkrankten Endothelzellen einschließlich der darunterliegenden Descemet-Membran entfernt und durch eine Descemet-Membran mit gesundem Hornhautendothel eines Spenders ersetzt werden. Vorteile dieser minimalinvasiven Operationstechnik gegenüber der konventionellen perforierenden Hornhauttransplantation bestehen in der vergleichsweise schnellen Erholung der Sehschärfe, nämlich Tage bis Wochen anstatt Monate bis Jahre, und dem geringeren Trauma.

In diesem Zusammenhang ist aus der US 2020/0170786 A1 ein artifizielles Augenhornhauttransplantat bekannt. Das beschriebene Hornhautimplantat verfügt über einen zentralen, transparenten Bereich mit einem Durchmesser von etwa 4 mm und eine diesen zentralen Bereich umgebende Einfassung. Der zentrale Bereich des Hornhauttransplantat wird vorzugsweise derart linsenförmig ausgebildet, dass Fehler bezüglich des Sehvermögens ausgeglichen werden können. Die Einfassung ist entweder ebenfalls transparent oder aber lichtundurchlässig. Wesentlich an der beschriebenen technischen Lösung ist, dass im Bereich der Einfassung ein netzartiger Bereich vorgesehen ist, der das Einwachsen von Zellen durch das Netz ermöglichen und so die Anhaftung des Implantats an die Augenhornhaut verbessern soll.

Weiterhin ist aus der WO 2010/083173 A2 ein Implantat, das auf der Rückseite der Augenhornhaut befestigbar ist, bekannt. Das beschriebene Implantat verfügt über eine hydrophobe Membran, die während der Implantation an die Hornhautrückwand angeklebt wird. Die Membran soll die Funktion einer Fusionsbarriere für Flüssigkeit übernehmen und die Gefahr des Aufquellens der Hornhaut aufgrund von eindringender Flüssigkeit verhindern oder zumindest verringern. Problematisch an der beschriebenen Membran ist, dass durch das eingebrachte Material eine Diffusionsbarriere erzeugt wird, die zu einer Beeinträchtigung der Dehydrierungsfunktion des Endothelzellen und somit zu einer Störung des Wasserhaushaltes der Hornhaut führen kann. Im Übrigen besteht bei dem für die Membran gewählten Material die Gefahr, dass unerwünschte Immunreaktionen hervorgerufen werden.

Bei Einsatz der bekannten Implantate besteht das Risiko, dass die Endothelzellen ihre Dehydrierungsfunktion verlieren, sodass es zu Wassereinlagerungen und damit einer Trübung der Hornhaut kommen kann. In diesem Zusammenhang stellt es daher eine Herausforderung für die Entwicklung von Implantaten für den Bereich der Augenhornhaut dar, dass die natürlichen Funktionen in der Augenhornhaut nicht oder nur möglichst wenig negativ beeinflusst werden und ein hohes Maß für die optische Transparenz sichergestellt wird. Weiterhin stellt es teilweise ein Problem dar, dass die in das Auge eingebrachten Implantate während und teilweise auch noch nach erfolgter Operation in ihrer Position fixiert werden müssen. Hierzu werden gezielt dehnbare Gas- oder Luftballone in die Augenhöhle eingebracht, die das jeweilige Implantat in der gewünschten Position gegen die Hornhautrückwand drücken. Aufgrund dieser Maßnahme werden die verwendeten Implantate allerdings einem zusätzlichen Druck ausgesetzt, der teilweise zu einer Schädigung der Endothelschicht, insbesondere zu einer Verringerung der Anzahl intakter Endothelzellen, führt.

Ausgehend von den aus dem Stand der Technik bekannten Implantaten, die die Augenhornhaut eines Menschen oder eines Tieres zumindest teilweise ersetzen, sowie den zuvor geschilderten Problemen liegt der Erfindung die Aufgabe zu Grunde, ein Implantat anzugeben, das die Behandlung eines Patienten mittels einer Hornhautrückwand-Transplantation (DMEK) unterstützt. Das anzugebende Implantat sollte hierbei die Möglichkeit bieten, eine Descemet-Membran und/oder Endothelzellen möglichst einfach, sicher und positionsgenau bei gleichzeitig verringertem Risiko von unerwünschten Immunreaktionen an der Hornhautrückwand zu befestigen. Im Weiteren sollte das für eine Behandlung verwendete Hornhauttransplantat, insbesondere die Endothelschicht, vor einer Beschädigung, vor allem durch einen während der Operation in die Augenhöhle eingebrachten Luft- oder Gasballon, geschützt werden und so der Untergang von Endothelzellen im transplantierten Spendergewebe verhindert oder zumindest reduziert werden. Darüber hinaus sollte sichergestellt werden, dass das Implantat eine Wasserbarriere bildet und die erforderliche Dehydrierung der Hornhaut realisiert oder ermöglicht und außerdem eine ausreichende Nährstoffversorgung in der Hornhaut erfolgen kann.

Das anzugebende Implantat sollte ferner auf vorteilhafte Weise zur Behandlung von kornealen Endothelpathologien, wie der Fuchs-Endotheldystrophie oder der pseudophaken bullösen Keratopathie, einsetzbar sein. Weiterhin sollte es möglich sein, auf oder in dem anzugebenden Implantat die benötigten Endothelzellen anzuziehen, sodass lediglich eine möglichst geringe Menge von Spendermaterial zur Realisierung der zuvor genannten Operationen erforderlich ist. Von besonderer Bedeutung ist es, dass durch das anzugebende Implantat die Transparenz der Augenhornhaut nicht oder nur geringfügig gegenüber der natürlichen Situation beeinträchtigt wird. Außerdem sollte Beachtung finden, dass das anzugebende Implantat vergleichsweise kostengünstig, prozesssicher, reproduzierbar und mit der erforderlichen Genauigkeit herstellbar ist.

Eine Aufgabe besteht somit auch darin, ein geeignetes Verfahren anzugeben, mit dem ein geeignetes Implantat, das die zuvor beschriebenen Eigenschaften aufweist, herstellbar ist.

Die zuvor beschriebene Aufgabe wird mit einer Stützmembran für ein Implantat gemäß Anspruch 1 und mit einem eine Stützmembran aufweisenden Implantat nach Anspruch 13 gelöst. Ein Verfahren zur Herstellung einer Stützmembran für ein Implantat, das die vorstehende Aufgabe löst, ist im Anspruch 14 angegeben. Vorteilhafte Ausführungsformen der Erfindung sind Gegenstand der abhängigen Ansprüche und werden in der folgenden Beschreibung unter teilweiser Bezugnahme auf Figuren näher erläutert.

Die Erfindung betrifft eine Stützmembran für ein Implantat zur Behandlung von Endothelerkrankungen einer Augenhornhaut eines Menschen oder eines Tieres mit einem Substrat, das für eine Befestigung an einer Rückwand der Augenhornhaut geeignet ist. Erfindungsgemäß zeichnet sich die Stützmembran dadurch aus, dass das Substrat drei übereinander angeordnete Schichten aufweist, von denen eine mittlere Schicht in Form einer Folie ausgebildet ist und über einen Rand verfügt, der zur Verankerung in der Augenhornhaut geeignet ist und dass beidseitig der mittleren Schicht wenigstens bereichsweise auf dieser Schicht äußere Schichten angeordnet sind, die jeweils eine poröse, flüssigkeitsdurchlässige Faserstruktur aufweisen. Erfindungsgemäß wird somit eine Stützmembran mit einem Schichtaufbau als Gerüststruktur zur Verfügung gestellt, die über wenigstens drei übereinander angeordnete Schichten verfügt. Wesentlich hierbei ist, dass eine mittlere Schicht in Form einer Folie ausgebildet ist, also ein vergleichsweise dünnes, homogenes Flächengebilde darstellt, das bevorzugt eine Flüssigkeits-, insbesondere Wasserbarriere bildet und somit den Durchtritt von Flüssigkeit bzw. Wasser verhindert oder zumindest erschwert. Da diese mittlere Schicht als Teil der erfindungsgemäß ausgeführten Stützmembran zumindest mittelbar an der Rückwand der Augenhornhaut eines Patienten befestigbar ist, wird nach erfolgter Implantation der Stützmembran, die bevorzugt gemeinsam mit Endothelzellen und/oder einer Descemet-Membran eines Spenders erfolgt, das Risiko eines durch eindringende Flüssigkeit verursachten Aufquellens der Hornhaut ausgeschlossen oder zumindest minimiert. Auf vorteilhafte Weise handelt es sich bei der mittleren Schicht um eine wasserabweisende Schicht, wobei es von besonderem Vorteil ist, wenn für diese Schicht ein Material verwendet wird, das sich innerhalb einer vorgebbaren Zeitspanne nach erfolgter Transplantation im Körper des Patienten auflöst bzw. vom Körper abgebaut werden kann.

Grundsätzlich handelt es sich bei der erfindungsgemäßen Stützmembran um ein Implantat, das einen multifunktionalen Schicht- oder Kompositaufbau aufweist und das bevorzugt bei der Behandlung der Fuchs-Endotheldystrophie oder der pseudophaken bullösen Keratopathie einsetzbar ist.

Die beiden äußeren Schichten, die zumindest bereichsweise auf gegenüberliegenden Seiten der mittleren Schicht flächig angeordnet sind, sind porös, strukturiert und/oder hydrophil und insbesondere zur Medienversorgung geeignet und verfügen bevorzugt über Fasern, die gerichtet oder ungerichtet angeordnet sind. Vorzugsweise bilden die Fasern ein Fasergewirk oder Faservlies. Gemäß einer speziellen Weiterbildung der Erfindung sind die Fasern derart ausgeführt und angeordnet, dass einerseits Flüssigkeit durch diese Schicht geleitet werden kann und andererseits ein Zellwachstum in oder an zumindest einer der äußeren Schichten möglich ist, insbesondere um die Haftfähigkeit der Stützmembran an einer Augenhornhaut zu verbessern.

In einer speziellen Ausführungsform ist die Stützmembran zumindest annähernd rund oder oval ausgebildet und verfügt über einen maximalen Durchmesser von 9 ± 4 mm. Von besonderem Vorteil ist es, wenn die Stützmembran zumindest bereichsweise gewölbt ist und eine maximale Dicke von 100 µm nicht überschreitet.

In einer speziellen Weiterbildung der Erfindung verfügt die mittlere und/oder wenigstens eine der äußeren Schichten über ein biokompatibles und/oder im Körper eines Menschen oder Tieres auflösbares oder abbaubares Polymer. Bevorzugt handelt es sich somit um ein sogenanntes Biopolymer, das einerseits über die erforderliche Körperverträglichkeit verfügt und sich andererseits über einen gewissen Zeitraum, der in Abhängigkeit der Eigenschaften des jeweils ausgewählten Biopolymers variieren kann, auflöst oder im Körper des Patienten abgebaut wird. Im Weiteren ist es von Vorteil, wenn die mittlere Schicht hydrophob und wenigstens eine der äußeren Schichten hydrophil ist. Generell ist es denkbar, dass die mittlere und die beiden äußeren Schichten aus dem gleichen Material oder aber aus unterschiedlichen Materialien hergestellt sind. Alternativ oder ergänzend ist es ferner denkbar, dass als Material für die mittlere und/oder wenigstens eine der äußeren Schichten ein Polymer verwendet wird, dass permanent im Körper des Patienten verbleibt, sich also nicht oder erst nach vergleichsweise langer Zeit auflöst.

Gemäß einer weiteren bevorzugten Ausgestaltung ist vorgesehen, dass wenigstens eine der äußeren Schichten ein ungerichtetes Fasergewirk oder Faservlies aufweist. Auf vorteilhafte Weise handelt es sich bei den verwendeten Fasern um Mikro- oder Nanofasern. Mit dieser Ausführungsform wird eine poröse, flüssigkeits-, insbesondere wasserpermeable Mikro- oder Nanofaserstruktur zumindest auf der Oberfläche der erfindungsgemäßen Stützmembran erzeugt.

Im Weiteren ist es denkbar, dass die mittlere und/oder wenigstens eine der äußeren Schichten wenigstens ein Polyester, Polycaprolacton, Polyactid, Polysaccharid, Hyaluron-Derivat und/oder zumindest ein Komposit, Copolymer und/oder Blendsystem der vorgenannten Materialien aufweist. Weiterhin ist es von Vorteil, wenn die mittlere Schicht wenigstens ein lipophiles oder amphophiles Additiv aufweist. Der Zusatz eines lipophilen Additivs bietet hierbei den Vorteil, dass in der als Barriereschicht dienenden mittleren Schicht ein Additiv zur Entquellung, etwa ein Dextran und/oder eine Salz wie zum Beispiel Natriumchlorid, vorgesehen ist.

Im Übrigen ist es denkbar, dass der Stützmembran, insbesondere wenigstens einer der äußeren Schichten, ein Additiv zugesetzt wird, das über einen gewissen Zeitraum im Körper des Patienten freigesetzt wird. Vorzugsweise wird hierfür als Additiv zumindest ein Protein, insbesondere ein Membranprotein, verwendet, das eine über einen längeren Zeitraum anhaltende entquellende Wirkung auf die Hornhaut hat. Denkbar ist auch der Zusatz von Hyalonsäure und/oder Dexpanthenol als Additiv in der äußeren Schicht. Im Übrigen ist es denkbar, dass als Additiv ein Wirkstoff zur Unterstützung eines Heilungsprozesses und/oder zur Minimierung von Abstoßungsreaktionen in wenigstens einer der äußeren Schichten angeordnet ist. Als mögliche Wirkstoffe kommen hier etwa Ophthalmika, Acetylsalicylsäure, Acetylcystein und/oder Vitamin A in Frage. Von besonderem Vorteil ist es, wenn die Auswahl eines Wirkstoffes derart erfolgt, dass eine retardierte, zeitversetzte Abgabe des Wirkstoffes, der als Additiv in wenigstens eine der äußeren Schichten der Stützmembran eingebettet ist, ermöglicht wird. Ebenso stellt es einen Vorteil dar, wenn es sich bei einem in der Stützmembran, insbesondere in wenigstens einer der äußeren Schichten, angeordneten Additiv um einen Wirkstoff handelt, der zur Förderung der Endothelialisierung gezielt Wachstumsfaktoren, zum Beispiel VEGF-Antagonisten, Aminothiol, Mercaptimin, Cenegermin und/oder Kortikosteroide, abgibt.

In einer weiteren besonderen Ausführungsform der Erfindung verfügt wenigstens eine der Schichten der Stützmembran, insbesondere die mittlere Schicht, über einen Farbstoff, etwa Trypanblau, der die Sichtbarkeit und Handhabbarkeit der Stützmembran während einer Operation verbessert und zu einer Verringerung der lokal auftretenden Belastungen während eines Eingriffs führt. Vorzugsweise wird ein Farbstoff gewählt, der innerhalb weniger Stunden im Körper des Patienten freigesetzt wird. Eine Stützmembran gemäß der Erfindung verfügt über eine mittlere Schicht, mit einem Rand, der zur Verankerung in der Augenhornhaut geeignet ist.

Damit eine geeignete Befestigung der Stützmembran an der Rückseite einer Augenhornhaut erfolgen kann, ist bevorzugt im Randbereich der mittleren Schicht eine Ankerstruktur vorgesehen, die auf vorteilhafte Weise eine Anhaftung der Stützmembran an der Augenhornhaut mittels geeigneter Haken ermöglicht. Im Hinblick auf die Operation, während der eine erfindungsgemäß ausgeführte Stützmembran in das Auge eines Patienten implantiert wird, stellt die Ankerstruktur eine Fixierung der Stützmembran dar, sodass die Zeitspanne, innerhalb der ein Luft- oder Gasballon benötigt wird, um die Stützmembran vorzugsweise mit den daran befestigten Endothelzellen und/oder einer Descemet-Membran an die Rückwand der Augenhornhaut zu drücken, zumindest verkürzt werden kann. Dies ist von großem Vorteil, da der während der Operation üblicherweise benötigte Luft- oder Gasballon die Versorgung der Endothelzellen mit Kammerwasser (Nährflüssigkeit) beeinträchtigt und stets das Risiko vorhanden ist, dass Endothelzellen beschädigt werden oder absterben.

Im Weiteren ist es gemäß einer speziellen Weiterbildung denkbar, dass die Stützmembran, insbesondere die mittlere Schicht im Bereich ihres Randes, der der Verankerung der Stützmembran in der Hornhaut dient, über Elemente verfügt, die eine wenigstens teilweise Verstärkung und/oder Magnetisierung der Stützmembran ermöglichen. In diesem Zusammenhang kann es von Vorteil sein, im Randbereich der mittleren Schicht, der etwa über eine kreisförmige, ovale, unregelmäßige oder sternförmige Umfangslinie verfügt, ein Polyamid, insbesondere in Form von Polyamidfäden, und/oder Kohlenstoffasern zur Verstärkung vorzusehen.

Vorzugsweise verfügt die Stützmembran, insbesondere der Rand der mittleren Schicht, über magnetische Partikel, wobei hierunter sowohl ferromagnetische, diamagnetische und paramagnetische Partikel, insbesondere metallhaltige Partikel, zu verstehen sind. Bevorzugt sind in diesem Randbereich Gold, Tantal, Silber, Platin und/oder Tetraferroplatin angeordnet. Auf vorteilhafte Weise wird eine Magnetisierung der Stützmembran, insbesondere des Randes der mittleren Schicht, durch eine wenigstens bereichsweise PVD-Besputterung mit Gold, Tantal, Silber, Platin und/oder Teraferroplatin realisiert. Besonders geeignet ist das Vorsehen von mit zumindest einem der zuvor genannten Materialien besputterten Kohlenstofffasern. Eine Magnetisierung der Stützmembran, insbesondere im Bereich des äußeren Randes der mittleren Schicht, trägt zu einer besseren Anhaftung der Stützmembran mit den daran oder darauf vorgesehenen Materialien und/oder weiteren Schichten bei. Hierbei sind geeignete Magnetkräfte zur verbesserten Fixierung bzw. Anhaftung der Stützmembran an der Augenhornhaut vorzugsweise durch Einsatz eines speziellen Magnetgegenpols, etwa einer Kontaktlinse und/oder einer Brille mit eingearbeitetem Neodymring oder Magnetband erzeugbar.

Auf vorteilhafte Weise werden die beiden äußeren Schichten gleich ausgeführt, sodass eine beidseitig implantierbare Stützmembran erzeugt wird.

Im Weiteren ist gemäß einer besonderen Ausführungsform vorgesehen, dass die Stützmembran, insbesondere wenigstens eine der äußeren Schichten, eine Wölbung aufweist oder derart ausgeführt ist, dass sie nach erfolgter Implantation gewölbt ist. Darüber hinaus ist es denkbar, dass die mittlere und/oder wenigstens eine der äußeren Schichten der Stützmembran über wenigstens ein Element aus einer Stoffgruppe verfügt, die Färbemittel, Wachstumsfaktoren, Antibiotika, Virostatika, Hormone und Immunsuppressiva enthält. Auf vorteilhafte Weise ist die mittlere Schicht transparent für sichtbares Licht, sodass insgesamt eine möglichst transparente Stützmembran erzeugt wird.

Zumindest eine der äußeren Schichten wird bevorzugt zumindest teilweise mittels Elektrospinnen erzeugt. Hierbei ist es möglich, gerichtete oder ungerichtete Fasergewirke oder Faservliese zu erzeugen. Fasergewirke mit zufällig organisierten, ungerichteten Fasern bewirken eine vergleichsweise geringe Transparenz. Daher kommen gemäß einer speziellen Weiterbildung Fasergewirke mit gerichteten Fasern zum Einsatz, die sich durch einen signifikant höheren Grad der Transparenz auszeichnen. Besonders bevorzugt werden hierbei Fasergewirke verwendet, die aus gerichteten Nanofasern erzeugt werden. Spektralanalysen haben in diesem Zusammenhang gezeigt, dass Fasergewirke mit gerichteten Nanofasern im Vergleich zu Fasergewirken mit ungerichteten Nanofasern eine doppelt so hohe Transparenz aufweisen.

In einer weiteren Ausführungsform ist hierzu vorgesehen, dass die Fasern eines Fasergewirks parallel und/oder von einem zentralen Punkt oder Bereich strahlenförmig nach außen gerichtet angeordnet sind. Im letztgenannten Fall nimmt der Abstand der Fasern daher von innen nach außen zumindest leicht zu.

Als ganz besonders geeignet erscheint es, wenn wenigstens eine der äußeren Schichten strahlenförmig von innen nach außen verlaufende Fasern, insbesondere Kohlenstoffasern, aufweist, auf die wenigstens bereichsweise Polybutylensuccinat (PBS) aufgebracht ist. Verfügt eine erfindungsgemäß ausgebildete Stützmembran über derartig gestaltete äußere Schicht entspricht die Transparenz der Stützmembran im Wellenlängenbereich des sichtbaren Lichts zumindest annähernd der der nativen Hornhaut.

Eine weitere besondere Ausgestaltung der Erfindung sieht vor, dass auf wenigstens einer der äußeren Schichten bereichsweise eine Descemet-Membran eines Spenders angeordnet ist. In diesem Fall ergibt sich die Möglichkeit, mithilfe einer erfindungsgemäß ausgeführten Stützmembran eine Descemet-Membran temporär oder permanent zu ersetzen wobei die Zeitspanne, die sich für die Nutzung ergibt, durch geeignete Auswahl von Materialien einstellbar ist und/oder von der Art und Anzahl der durchgeführten Operation abhängt. Alternativ oder in Ergänzung ist es denkbar, dass auf oder in wenigstens einer der äußeren Schichten Endothelzellen angeordnet sind. Auf vorteilhafte Weise ist die Stützmembran multifunktionell, in Bezug auf den Schichtaufbau symmetrisch und damit unabhängig von ihrer Lage implantierbar. Besonders bevorzugt ist eine erfindungsgemäße Stützmembran im Übrigen rollbar, sodass diese etwa in aufgerolltem Zustand in ein Auge einbringbar und daraufhin im Auge ausrollbar ist.

Die Erfindung betrifft somit sowohl eine Stützmembran als auch ein Implantat mit einer Stützmembran, die erfindungsgemäß und gemäß zumindest einer der zuvor beschriebenen Ausführungsformen ausgeführt und daher zum wenigstens teilweisen Ersatz einer Augenhornhaut, insbesondere der Descemet-Membran und/oder von Endothelzellen geeignet ist.

Im Weiteren betrifft die Erfindung auch ein Verfahren zur Herstellung einer Stützmembran für ein Implantat zur Behandlung von Endothelerkrankungen einer Augenhornhaut eines Menschen oder eines Tieres mit einem Substrat mit den Schritten:
- Erzeugen einer äußeren porösen, wasserdurchlässigen Schicht aus einem ersten Material im Wege eines Elektrospinnvorgangs und Aufbringen der ersten äußeren Schicht auf eine gewölbte Form,
- Aufbringen einer homogenen, folienartigen mittleren Schicht auf die äußere Schicht durch Aufsprühen eines zweiten Materials auf die erste äußere Schicht sowie
- Erzeugen einer zweiten äußeren porösen, wasserdurchlässigen Schicht aus dem ersten Material im Wege eines Elektrospinnvorgangs und Aufbringen der zweiten äußeren Schicht auf die mittlere Schicht.

Die zweite äußere Schicht wird somit auf die der ersten äußeren Schicht gegenüberliegenden Seite der mittleren Schicht wiederum flächig aufgebracht. Es ist nicht zwingend erforderlich, dass die oben genannten Verfahrensschritte in der angegebenen Reihenfolge durchgeführt werden. Ebenso ist es in diesem Zusammenhang denkbar, wenigstens zwei der Verfahrensschritte zu kombinieren, beispielsweise durch Nutzung verschiedener Düsen während des Elektrospinnens, durch die unterschiedliche Materialien austreten bzw. ausgesprüht werden. Das jeweils für die äußeren Schichten verwendete erste Material sowie das für die mittlere Schicht verwendete zweite Material kann zumindest teilweise identisch oder unterschiedlich ausgewählt werden. Als geeignetes Material kommen hierbei wiederum bevorzugt die Stoffe und Stoffzusammensetzungen, die zuvor in der Beschreibung des erfindungsgemäß ausgeführten Stützmembran offenbart sind, zum Einsatz. Besonders bevorzugt wird das erste und/oder das zweite Material ausgewählt aus einer Gruppe, zu der Polycaprolacton, Polyactid, Polysaccarid, Gelatine, Alginat, Hyaluron-Derivat und Komposite, Copolymere und Blendsysteme der vorgenannten Materialien gehören.

Gemäß einer speziellen Weiterbildung wird die mittlere Schicht und damit das zweite Material mit wenigstens einem lipophilen oder amphiphilen Additiv, beispielsweise einem Fett, einer Fettsäure, insbesondere Myristinsäure, einem Phospholipid, einem Phosphatidylcholin, insbesondere Lecitin und/oder einem Lipopolysaccarid versehen. Ebenso ist es denkbar die mittlere Schicht bzw. das zweite Material mit einem Farbstoff, insbesondere Trypanblau, zu ergänzen. Im Weiteren ist es denkbar, wenigstens einer der Schichten bzw. den zu deren Erzeugung verwendeten Material wenigstens ein funktionales Additiv, wie etwa einen Wachstumsfaktor, insbesondere VEGF (Vascular Endothelial Growth Factor), ein Antibiotikum, ein Virostatikum, insbesondere Ganciclovir, ein Antimyotikum, ein Corticosteroid, insbesondere Difluprednate, und/oder ein Immunsupressivum, insbesondere Cyclosporin-A, zuzugeben.

Auf vorteilhafte Weise ist hierbei vorgesehen, dass die äußeren Schichten im Wege eines Elektrospinnvorgangs hergestellt und so eine Faserstruktur, insbesondere eine Nano- oder Mikrofaserstruktur bzw. ein Faservlies mit gerichteten oder ungerichteten Nano- oder Mikrofasern hergestellt wird. Die erste äußere Schicht wird bevorzugt mithilfe eines Elektrospinnvorgangs erzeugt und auf ein pilzförmiges Target aufgebracht, sodass eine erste äußere Schicht mit einer gewölbten Form erzeugt wird. Somit lässt sich durch Auswahl eines Targets mit einer speziell geformten Oberfläche eine erste äußere Schicht mit einer gewünschten Form herstellen.

Daraufhin wird die mittlere Schicht bevorzugt erzeugt, indem das zur Herstellung einer Folie verwendete Material auf die erste äußere Schicht, insbesondere im Wege eines Airbrushverfahrens, aufgesprüht wird.

Die zweite äußere Schicht wird wiederum im Wege eines Elektrospinnvorgangs erzeugt und auf die mittlere Schicht aufgebracht. Die Oberfläche der so erzeugten, dreischichtigen Stützmembran verfügt auf zwei gegenüberliegenden Seiten über eine poröse, flüssigkeits- oder wasserpermeable Nano- oder Microfaserstruktur, die aufgrund dieser Eigenschaft eine Nährstoffversorgung einer Descemet-Membran durch seitlich in wenigstens eine der äußeren Schichten ein fließendes Kammerwasser ermöglicht. Die mittlere, folienartige Schicht erschwert hingegen einen Flüssigkeits- bzw. Wasserdurchtritt und stellt so nach erfolgter Implantation sicher, dass ein unerwünschtes Aufquellen der Augenhornhaut verhindert oder das Risiko, das es zu einem Aufquellen der Augenhornhaut kommt, zumindest minimiert wird.

Im Folgenden wird die Erfindung ohne Beschränkung des allgemeinen Erfindungsgedankens anhand spezieller Ausführungsbeispiele unter Bezugnahme auf die Figuren näher erläutert. Dabei zeigen:
- Fig. 1:: Schematische Darstellung des anatomischen Aufbaus der einzelnen Hornhautschichten des menschlichen Auges mit normalen Hornhautbedingungen;
- Fig. 2:: Schematische Darstellung des anatomischen Aufbaus der einzelnen Hornhautschichten des menschlichen Auges mit einer aufgrund der Augenkrankheit Keratokonus veränderten, kegelförmig gewölbten Hornhaut;
- Fig. 3:: Querschnittsdarstellung einer erfindungsgemäß ausgeführten Stützmembran unter unterschiedlichen Belastungen
- Fig. 4:: Draufsicht auf eine der äußeren Schichten einer erfindungsgemäß ausgeführten Stützmembran;
- Fig. 5:: stark vergrößerte schematische Darstellung eines Ausschnitts einer der äußeren Schichten einer erfindungsgemäß ausgeführten Stützmembran sowie
- Fig. 6:: Ablaufschema einer Hornhautrückwand-Transplantation (DMEK).

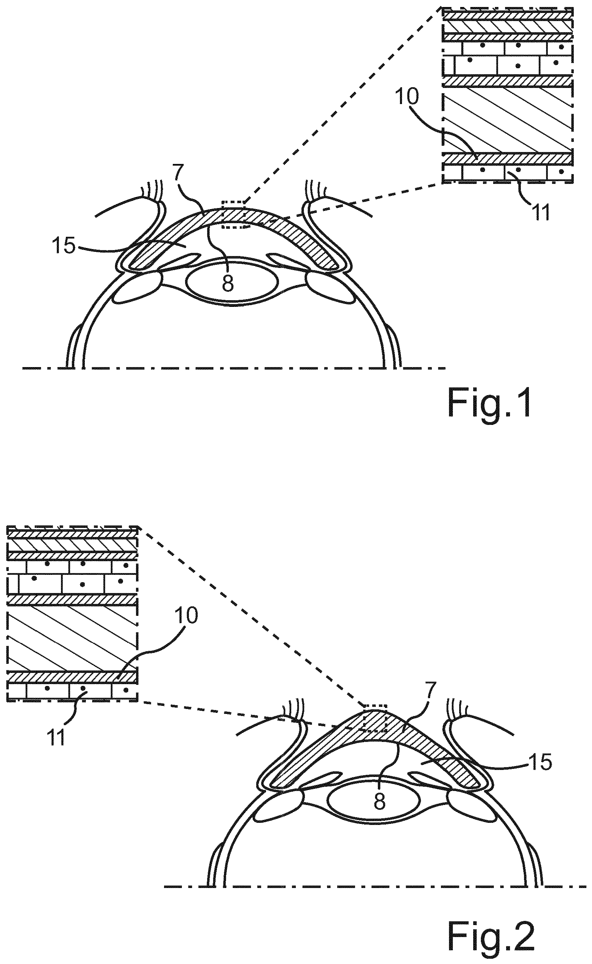

Fig. 1 zeigt in einer schematischen Darstellung den anatomischen Aufbau der einzelnen Hornhautschichten des gesunden menschlichen Auges. Im Vergleich hierzu zeigt Fig. 2 eine aufgrund der Augenkrankheit Keratokonus veränderte Augenhornhaut mit einer kegelförmigen Vorwölbung.

Die gesunde Augenhornhaut 7 ist der üblicherweise klare, von Tränenflüssigkeit benetzte, gewölbte vordere Teil der äußeren Augenhaut und leistet einen Großteil der Lichtbrechung. Sie stellt hierbei den frontalen Abschluss des Augapfels dar.

Die menschliche Hornhaut 7 besteht aus sechs Schichten, nämlich der Epithelschicht, der Bowman-Schicht, dem Stroma, der Descemet-Membran 10, der Endothelzellenschicht 11 sowie der Dua-Schicht, die seit dem Jahr 2013 als eine etwa 15 µm dicke Schicht zwischen Stroma und der Descemet-Membran 10 definiert ist und aus 5 bis 8 Lamellen von Kollagen-Typ-1-Bündeln besteht. Da die Erfindung eine Stützmembran 1, ein Implantat 2 mit einer Stützmembran 1 sowie ein Verfahren zur Herstellung einer Stützmembran 1 betrifft, die zur Behandlung von Endothelerkrankungen eingesetzt wird, beschränkt sich die folgende Beschreibung auf die Endothelschicht 11 und die Descemet-Membran 10.

Die Oberfläche der Endothelschicht 11 bildet auf der Rückseite der Hornhaut 7 die Oberfläche und besteht aus 5 bis 6 Schichten von Epithelzellen. Im Mittel ist diese Schicht etwa 40 bis 60 µm dick. Die Descemet-Membran 10 stellt hingegen die dickste Basalmembran im menschlichen Körper dar. Sie ist zur Zeit der Geburt etwa 3 µm und im Erwachsenenalter etwa 8 bis 10 µm dick, die Dicke der Descemet-Membran 10 nimmt somit im Laufe des Lebens zu.

Die Descemet-Membran 10 ist in verschiedene Schichten unterteilt, von denen es die hintere Schicht ist, die im Leben ständig an Dicke durch Auflagerungen von Endothelkollagenen zunimmt. Es wird vermutet, dass die Descemet-Membran 10 ein Ausscheidungsprodukt des Endothels ist. Im Übrigen kompensiert sie die mit zunehmendem Alter abnehmende Pumpleistung des Endothels 11. Die Descemet-Membran 10 ist durchsichtig, homogen und besteht hauptsächlich aus Kollagenfasern des Typs VIII und Laminin. Die Descemet-Membran 10 übernimmt eine Schutzfunktion für die Endothelzellen in Bezug auf Infektionen, mechanische und chemische Verletzungen sowie enzymatische Zerstörung.

Das Endothel 11 besteht aus einer Einzelschicht abgeflachter, hexagonaler Zellen, die etwa 5 µm dick sind und einen Durchmesser von etwa 20 µm aufweisen. Die Zelldichte beträgt bei jungen, erwachsenen Menschen ungefähr 3500 Zellen/mm², wobei die Anzahl der Endothelzellen mit fortschreitendem Alter und unabhängig von Erkrankungen stetig abnimmt, sodass die Zelldichte im hohen Alter noch etwa 2000 Zellen/mm² beträgt. Die vordere Seite der Endothelzellen grenzt flach an die Descemet-Membran 10, während sich die hintere Seite an der Grenze zur Vorderkammer des Auges befindet. Eine der wichtigsten Aufgaben des Endothels 11 ist es, für eine Dehydrierung der Hornhaut 7 zu sorgen und so deren Transparenz aufrechtzuerhalten. Eine weitere Aufgabe besteht in der Synthese bestimmter Komponenten der Descemet-Membran 10 und der Regulierung des Austausches von Stoffwechselprodukten zwischen Kammerwasser und Stroma. Insbesondere wird von den Endothelzellen das durch den Stoffwechselvorgang eindringende Kammerwasser aus der Hornhaut 7 herausgepumpt.

Kommt es zu einer Erkrankung des Hornhautendothels 11, beispielsweise der Fuchs-Endotheldystrophie oder der pseudophaken bullösen Keratopathie, ist das Hornhautendothel 11 in vivo nicht in der Lage, sich zu regenerieren. Insbesondere um einer Eintrübung der Hornhaut 7 entgegenzuwirken, ist es in diesen Fällen erforderlich, die innere Endothelschicht 11 der Hornhaut durch ein Transplantat zu ersetzen. Ebenso sind Erkrankungen bekannt, die die Descemet-Membran 10 schädigen, sodass Kammerwasser in die Hornhaut 7 eindringen kann und es zu einem Aufquellen und wiederum einer Eintrübung der Hornhaut 7 kommen kann. Zu diesen Augenkrankheiten gehört etwa der Keratokonus, eine der häufigsten Wölbungsanomalien der Hornhaut 7, bei der sich die Hornhaut 7 kegelförmig verformt und hierdurch bereichsweise immer dünner wird. Durch das fortschreitende Ausdehnen besteht sogar die Gefahr, dass die Descemet-Membran 10 einreißt. In diesem Zusammenhang zeigt Fig. 2 in einer schematischen Darstellung den anatomischen Aufbau eines Auges mit seinen Hornhautschichten, das an einem Keratokonus erkrankt ist. Im Vergleich zu dem in Fig. 1 dargestellten gesunden Auge fällt die kegelförmige Vorwölbung der Hornhaut 7 auf.

Je nach Ausmaß der oben genannten Erkrankungen ist es möglich, nicht die gesamte Hornhaut 7 auszutauschen, sondern nur einen Teil, nämlich insbesondere die Descemet Membran 10 mit dem Endothel 11, im Wege einer Hornhautrückwand-Transplantation (Descemet Membrane Endothelial Keratoplasty - DMEK) durch Spendermaterial zu ersetzen.

Bei der Hornhautrückwand-Transplantation (DMEK) handelt es sich um ein schonendes und minimal invasives Transplantationsverfahren, bei dem die erkrankten Endothelzellen einschließlich der darunterliegenden Descemet-Membran 10 entfernt und durch eine Descemet-Membran 10 mit gesundem Hornhautendothel 11 eines Spenders ersetzt werden. Da der weltweite Bedarf an Spendermaterial größer ist als die zur Verfügung stehenden Transplantate, werden zum Teil natürlich gewachsene Zellen auf Trägerschichten aufgebracht und diese in das Auge eines Patienten implantiert. Da Endothelzellen in vitro zur Proliferation befähigt sind, werden verschiedene Konzepte zum Tissue Engineering verfolgt, bei denen Zellen auf natürlich gewachsenen oder artifiziellen Trägermaterialien kultiviert werden. Nach erfolgter Kultivierung wird das erzeugte Endothel 11 gemeinsam mit dem Trägermaterial auf die endothelfreie Rückwand 8 der Hornhaut 7 des Patienten implantiert. Die hierbei als Trägermaterial verwendeten natürlich gewachsenen oder artifiziellen Membranen bestehen oftmals aus biologischen Polymeren, die sich durch eine hohe Biokompatibilität auszeichnen. Problematisch ist allerdings in vielen Fällen, dass durch die eingebrachten Materialien eine Diffusionsbarriere erzeugt wird, die zu einer Beeinträchtigung der Dehydrierungsfunktion der Endothelzellen und somit zu einer Störung des Wasserhaushaltes der Hornhaut 7 führen. Darüber hinaus kann es durch die eingebrachten Materialien zu unerwünschten Immunreaktionen kommen.

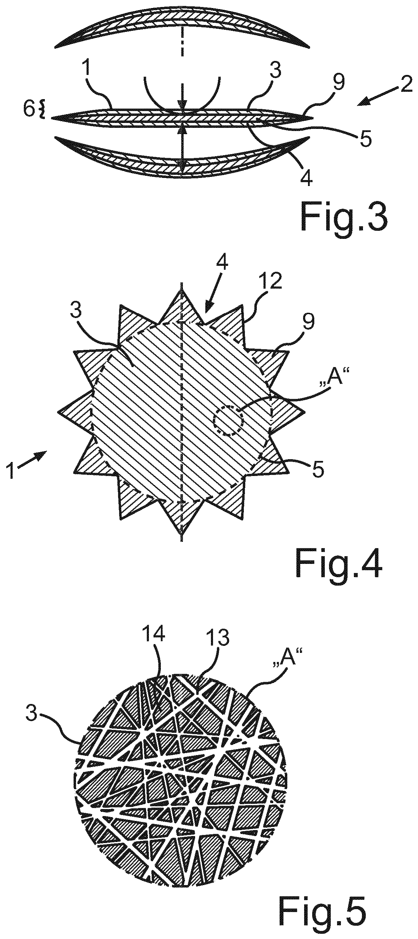

Die zuvor beschriebenen Probleme können mit einer erfindungsgemäß ausgeführten Stützmembran 1, wie sie in Fig. 3 in einer Schnittdarstellung in drei verschiedenen Belastungszuständen dargestellt ist, gelöst werden. Die Stützmembran 1 weist einen Schichtaufbau mit drei Schichten 3, 4, 5 auf, nämlich mit einer mittleren Schicht 5, die in Form einer Folie ausgebildet ist, auf der zu beiden gegenüberliegenden Seiten jeweils eine äußere Schicht 3, 4 angeordnet ist. Die mittlere Schicht 5 in Form einer Folie, beispielsweise aus biodegradierbarem Polyester, etwa Polycaprolacton oder Polyactid, bildet die mechanische Grundstruktur und gleichzeitig eine Barriereschicht, die einen Durchtritt von Wasser, insbesondere Kammerwasser, zumindest erschwert. Demgegenüber weisen die beiden äußeren Schichten 3, 5 ein Fasergewirk mit Nanofasern auf, sind porös und wasserpermeabel und ermöglichen daher einerseits den Transport von Nährstoffen durch die vorhandenen Poren und tragen andererseits zu einer verbesserten zellulären Adhärenz bei. Die äußeren Schichten 3, 4 verfügen über gerichtete Fasergewirke aus Nanofasern aus biodegradierbarem Polyester, etwa Polycaprolacton oder Polyactid. Im Weiteren ist die Stützmembran 1 derart ausgeführt, dass sie bei einer Kraftbeaufschlagung die ebenfalls in Fig. 3 dargestellten Formen mit einer Wölbung zu einer Seite aufweisen kann. Bevorzugt wird eine Stützmembran 1 mit einer bedarfsgerecht ausgeführten Wölbung hergestellt, sodass diese auf besondere Weise an die Hornhautrückwand anpassbar ist und, insbesondere auf der Stützmembran angeordnete Endothelzellen und/oder eine Descemet-Membran, vergleichsweise schnell an der Hornhautrückwand 8 anwächst.

Ergänzend zeigt Fig. 4 eine Draufsicht auf eine der äußeren Schichten 3 sowie auf den zur Verankerung in der Hornhautrückwand 8 vorgesehenen umlaufenden Rand 9 der mittleren Schicht 5 einer erfindungsgemäß ausgeführten Stützmembran 1. Da gemäß der hier beschriebenen Ausführungsform die beiden äußeren Schichten 3, 4 der Stützmembran 1 gleich ausgebildet sind, verfügt die Stützmembran 1 in Bezug auf den Schichtaufbau über eine symmetrische Struktur, sodass jede der beiden äußeren Schichten 3, 4 bei einer Implantation ohne Nachteil der Hornhautrückwand 8 zugewandt sein kann. Die zumindest nahezu kreisförmigen äußeren Schichten 3, 4, von denen in Fig. 4 nur eine sichtbar ist, sind porös und weisen ein Nanofasergewirk mit gerichteten Nanofasern auf. Zwischen den beiden äußeren Schichten 3, 4 ist eine mittlere Schicht 5 angeordnet, die entlang ihres Umfangs, der über den äußeren Umfang der äußeren Schichten 3, 4 hinausragt, einen Rand 9 mit Verankerungszacken aufweist, über die die Stützmembran 1 in der Augenhornhaut 7 eines Patienten verankert werden kann. Durch die gemäß der dargestellten Ausführungsform vorgesehenen Verankerungszacken 12 wird ein schnelles Einwachsen der Stützmembran 1 in die natürlichen Strukturen einer Augenhornhaut 7 gewährleistet. Aufgrund einer derartigen Verankerung kann der bei einer operativen Transplantation in die Augenhöhle üblicherweise eingebrachte Gas- oder Luftballon, der das Implantat mit der Descemet-Membran 10 und den Endothelzellen an die Rückwand 8 der Augenhornhaut 7 andrücken soll, bereits nach kurzer Zeit wieder aus dem Auge entfernt werden, sodass eine Beschädigung der implantierten Endothelzellen durch den Gas- oder Luftballon auf diese Weise verhindert oder zumindest verringert werden kann.

Zur Herstellung der beschriebenen äußeren Schichten 3, 4 bietet sich vor allem Elektrospinnen an. Bevorzugt werden hierbei die elektrogesponnenen Strukturen aus degradierbaren und biokompatiblen Materialien hergestellt. Der Vorteil besteht darin, dass verschiedene Arten von Hornhautzellen auf elektrogesponnenen Stützmembranen oder Gerüststrukturen wachsen.

Im Übrigen besteht eine Herausforderung bei der Herstellung von Hornhautimplantaten stets darin, ein möglichst hohes Maß an optischer Transparenz sicherzustellen. Elektrogesponnene, zufällig organisierte Fasern 13 weisen im Allgemeinen eine geringe Transparenz auf. Für den Ausschnitt "A" einer derart ausgeführten äußeren 3 Schicht ist in Fig. 5 eine stark vergrößerte schematische Darstellung gezeigt. Die durch Elektrospinnen hergestellte äußere Schicht 3 einer erfindungsgemäß ausgeführten Stützmembran 1 verfügt über ein Gewirk aus ungerichteten Nanofasern 13, wobei zwischen den einzelnen Nanofasern 13 Poren 14 gebildet werden. Die Schicht 3 verfügt somit über eine entsprechend poröse, flüssigkeits- bzw. wasserpermeable Struktur, durch die Nährmedium hindurchtreten kann.

Ein signifikant höherer Grad an Transparenz wird erreicht, sobald die Fasern 13 ausgerichtet sind. Der Vergleich der Transparenz mittels Spektralanalyse hat gezeigt, dass die Transparenz eines ausgerichteten Nanofaservlieses doppelt so hoch, wie die eines zufällig orientierten Nanofaservlieses ist. Eine besonders hohe Transparenz lässt sich allerdings nicht nur mit parallel ausgerichteten Fasern 13 erzielen, sondern vielmehr kann Elektrospinnen auch zur Herstellung von Fasern 13 verwendet werden, die von einem Zentrum ausgehend strahlenförmig angeordnet sind. Werden im Wege des Elektrospinnens die verschiedenen Fasern 13 strahlenförmig ausgerichtet und mit Polybutylensuccinat (PBS) beschichtet, lässt sich eine Schicht mit einer Transparenz, die der Transparenz der nativen Hornhaut im sichtbaren Wellenlängenbereich ähnlich ist, erzeugen.

Eine erfindungsgemäß ausgebildete Stützmembran 1 eignet sich auf besondere Weise zur Unterstützung einer Hornhautrückwand-Transplantation (DMEK), deren Vorteile gegenüber der konventionellen, perforierenden Hornhauttransplantation, bei der nicht eine einzelne Schicht, sondern die komplette Hornhaut 7 transplantiert wird, in der relativ schnellen Erholung der Sehschärfe, hier nämlich Tage bis Wochen anstatt Monate bis Jahre bei der perforierenden Hornhauttransplantation, und in dem geringeren Trauma während der Operation bestehen. Eine Vereinfachung dieser Operation könnte ferner dazu führen, dass diese nicht nur in hierauf spezialisierten Kliniken, sondern auch in anderen Augenkliniken durchgeführt werden würde.

Um das Anwachsen des Transplantats bei einer Teiltransplantation der hinteren Hornhaut 7 zu fördern, wird üblicherweise ein Gas- oder Luftballon in das Auge eingebracht, der das Transplantat an die Hornhautrückwand 8 andrückt. Da die Endothelzellen das Kammerwasser als Nährflüssigkeit benötigen, führt das Einbringen dieses Gas- oder Luftballons und der durch diesen verursachte Anpressdruck zu einem Absterben von Endothelzellen und hat einen Rückgang der Zelldichte der transplantierten Endothelzellen zur Folge. Dies kann zu einer Verschlechterung oder sogar einem Verlust der Dehydrierungsunktion der Endothelzellen führen, wodurch Wassereinlagerungen und damit eine Eintrübung der Hornhaut 7 verbunden ist.

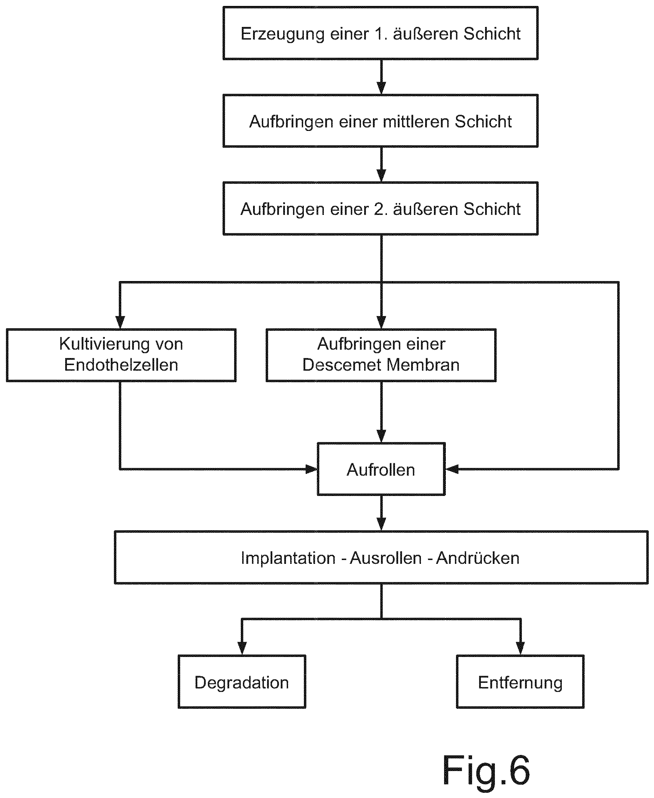

Zur Verdeutlichung der Operationstechnik sowie des Einsatzes und der Vorteile einer erfindungsgemäß ausgeführten Stützmembran 1 zeigt Fig. 6 in einem Ablaufschema verschiedene Schritte bei der Vorbereitung und Durchführung einer Hornhautrückwand-Transplantation (DMEK). Das verwendete Implantat 2 verfügt über eine erfindungsgemäß ausgeführte artifizielle Stützmembran 1, deren äußere Schichten durch Elektrospinnen eines Biopolymers hergestellt wurden. Als Biopolymer kommen hierbei wahlweise Stoffe ausgewählt aus einer Gruppe, die Polycaprolacton, Polyactid, Polysaccarid, Gelatine, Alginat, Hyaluron-Derivat sowie Komposite, Copolymere und Blendsysteme der vorgenannten Materialien enthält, zum Einsatz. Wie im Folgenden noch näher erläutert werden wird, wird zunächst eine erste äußere Schicht 3 durch Elektrospinnen hergestellt und in die gewünschte Form gebracht. Auf diese erste äußere Schicht 3 wird eine mittlere folienartige Schicht 5 durch Sprühen, Tauchen und/oder Drucken als Barriereschicht aufgebracht. Abschließend wird eine zweite äußere Schicht 4 durch Elektrospinnen hergestellt und auf die der ersten äußeren Schicht 3 gegenüberliegenden Seite der mittleren Schicht 5 aufgebracht. Wesentlich an diesem Herstellungsverfahren ist, dass bei der Herstellung ein Schichtaufbau durch eine Kombination komplementärer Technologien, wie dem Elektrospinnen einerseits und Beschichtungsverfahren, wie etwa Ultraschallsprühen, Schmelztauchen, Tauchen, Sprühen oder 3D-Drucken andererseits erzeugt wird. Auf vorteilhafte Weise lassen sich so bedarfsgerecht insbesondere Stützmembrane 1 mit nanofaserigen Oberflächeneigenschaften und mit einer dünnen Folienschicht mit spezieller Form herstellen. Durch derartige Stützmembrane 1 lässt sich vor allem die Durchführung der endothelialen Transplantation der Hornhaut 7 und das Anwachsverhalten des Endothels 11 und/oder der Descemet-Membran 10 verbessern, wobei ferner nützliche Additive, wie etwa Wachstumsfaktoren, Farbstoffe und/oder sonstige medizinisch wirksame Stoffe auf einfache Weise applizierbar sind. In diesem Zusammenhang wird darauf hingewiesen, dass der gemäß dem Fig. 6 geteigten Ausführungsbeispiel vorgesehene Schritt der Kultivierung von Endothelzellen lediglich optional ist, da bei der Realisierung der Erfindung generell auch Systeme bzw. Schichtaufbauten verwendet werden können, die keine Endothelzellen aufweisen.

Gemäß dem in Fig. 6 gezeigten Ablaufschema wird vor der eigentlichen Hornhautrückwand-Transplantation zunächst eine Stützmembran 1 für eine Spender-Descemet-Membran 10 oder als temporäres Ersatzgewebe für Hornhautendothelzellen erzeugt. Nach erfolgter Implantation wird durch diese Stützmembran 1, insbesondere durch ihre porösen, flüssigkeitsdurchlässigen äußeren Schichten 3, 4 die Versorgung der Endothelzellen mit Kammerwasser und damit mit Nährstoffen gewährleistet. Die gemäß der beschriebenen Ausführungsform erzeugte Stützmembran 1 ist transparent und verfügt über eine Dicke von 10 ± 5 µm.

Zur Herstellung der Stützmembran 1 wird in einem ersten Prozessschritt eine 3 ± 2 µm dicke Schicht 3 aus einem biodegradablem Polymer, hier aus einer speziellen Polymilchsäure (Poly(L-lactid acid (PLLA)), die bevorzugt mit Lecithin und Triethyl-O-acetylcitrat gemischt wird, durch Elektrospinnen hergestellt und auf ein pilzförmiges Target aufgebracht. Alternativ kann diese Schicht 3 auch aus einer Mischung, die Polycaprolacton und Gelatine enthält, erzeugt werden.

Diese Schicht 3 bildet später eine der beiden äußeren Schichten 3, 4 der erfindungsgemäßen Stützmembran 1. Während diese Schicht 3 auf das pilzförmige, elektrisch kontaktierbare Target aufgebracht wird, erhält sie die gewünschte Form mit einer spezifischen Wölbung.

In einem nächsten Prozessschritt wird im Airbrushverfahren eine Schicht 5 aus einem biodegradablem Polymer, hier aus einer speziellen Polymilchsäure (Poly(L-lactid acid (PLLA)), die bevorzugt mit Lecithin und Triethyl-O-acetylcitrat gemischt wird, auf die erste äußere Schicht 3 aufgesprüht und so eine Folie, also ein homogenes Flächengebilde, das den Durchtritt von Wasser erschwert, auf der ersten äußeren Schicht 3 erzeugt. Diese Schicht 5, die später die mittlere Schicht 5 der zu erzeugenden Stützmembran 1 bildet, enthält zusätzlich Trypanblau als Farbstoff. Vor dem Aufsprühen dieser mittleren Schicht 5 wird daher zunächst eine Lösung, die eine Polymilchsäure (Poly(L-lactid acid (PLLA)) und Trypanblau sowie bevorzugt auch Lecithin und Triethyl-O-acetylcitrat enthält hergestellt.

Auf diese mittlere Schicht 5 wird in einem weiteren Prozessschritt wiederum eine durch Elektrospinnen hergestellte 3 ± 2 µm dicke Schicht 4, die die zweite äußere Schicht 4 bildet, aus einem biodegradablem Polymer, hier aus einer speziellen Polymilchsäure (Poly(L-lactid acid (PLLA)), aufgebracht. Alternativ kann diese Schicht auch aus einer Mischung, die Polycaprolacton und Gelatine enthält, erzeugt werden.

In einem abschließenden Schritt erfolgt eine Aktivierung der drei Schichten 3, 4, 5 aufweisenden Stützmembran 1 mithilfe von Sauerstoffplasma.

Die Oberfläche der so hergestellten Stützmembran 1 weist auf beiden Seiten eine poröse, flüssigkeits- oder wasserpermeable Nanofaserstruktur auf, die eine Nährstoffversorgung der Descemet-Membran 10 und des Endothels 11 durch seitlich in die äußeren Schichten 3, 4 einfließendes Kammerwasser ermöglicht. Im Vorfeld der Operation wird auf eine der beiden äußeren Schichten 3, 4 die Spender-Descemet-Membran 10 aufgebracht und die Stützmembran 1 gemeinsam mit der darauf angeordneten Descemet-Membran 10 in das Auge des Patienten eingeführt. Alternativ ist es denkbar, dass die Stützmembran 10 und die Descemet-Membran 11 während der Operation nacheinander in die Vorderkammer des Auges eingeführt werden.

Im Weiteren ist es möglich, auf zumindest einer der beiden äußeren Schichten 3, 4 noch vor der Operation Hornhaut-Endothelzellen zu kultivieren, zumindest falls dies notwendig erscheint. Da beide äußeren Schichten 3, 4 gleich ausgeführt sind, wird auf vorteilhafte Weise sichergestellt, dass auch bei einer versehentlich falschen Orientierung des Implantats 2 während der Operation ein Erfolg sichergestellt ist.

Der in die mittlere Schicht 5 eingebrachte Trypanblau-Farbstoff dient der besseren Sichtbarkeit der Stützmembran 1 während der Operation. Alternativ oder ergänzend ist es denkbar, weitere Additive, insbesondere medizinisch wirksame Stoffe, in eine der drei Schichten 3, 4, 5 einzubringen, die dann über einen gewissen Zeitraum im Auge freigesetzt werden.

Die mittlere Schicht 5 der Stützmembran 1 verfügt im Übrigen über einen entlang des Umfangs angeordneten, durch Polyamidfäden verstärkten Rand 9, der eine zügige Verankerung der Stützmembran 1 in der Augenhornhaut 7 sicherstellt.

Darüber hinaus ist es denkbar, im Bereich des Randes 9 magnetische Materialien, beispielsweise in Form von Partikeln, vorzusehen, sodass aufgrund von magnetischen Kräften eine Immobilisierung der Stützmembran 1 an der Hornhaut 7 erfolgen kann, insbesondere eine sichere Anhaftung der Stützmembran an der Hornhautrückseite sichergestellt ist. Magnete, die geeignete magnetische Gegenpole darstellen, können beispielsweise in Kontaktlinsen oder einer Brille angeordnet sein. Durch diese Maßnahme wird wiederum sichergestellt, dass die Stützmembran 1 sicher in ihrer Position relativ zur Hornhautrückwand 8 fixiert ist und ein Anwachsen an der Hornhaut 7 begünstigt wird. Der Zeitraum, über den ein üblicherweise bei einer Hornhautrückwand-Transplantation im Auge angeordneter Gas- oder Luftballon im Auge verbleibt und auf das Implantat drückt, kann so zumindest erheblich verkürzt werden.

Während einer Hornhautrückwand-Transplantation wird gemäß der beschriebenen Ausführungsform eine Descemet-Membran 10, die auf einer Stützmembran 1 angeordnet ist, entsprechend den üblichen Operationstechniken in aufgerolltem Zustand in die Vorderkammer 15 des Auges eingebracht. Nach dem Ausrollen und Andrücken der Stützmembran 1 mit der darauf angeordneten Descemet-Membran 10 erfolgt der Verbleib an der Hornhautrückwand, 8 wobei die Anpassung an die Hornhautrückwand 8 durch die spezielle Form der Stützmembran 1, die insbesondere durch die Geometrie der mittleren Schicht 5 vorgegeben ist, begünstigt wird.

Ebenso ist es möglich, während einer Hornhautrückwand-Transplantation die Stützmembran 1 mit darauf angeordneten, kultivierten Endothelzellen entsprechend den üblichen Operationstechniken in die Vorderkammer einzubringen. Nach Ausrollen und Andrücken der Stützmembran mit den aufgebrachten Endothelzellen verbleibt dieses Implantat 2 an der Hornhautrückwand 8.

In Bezug auf die in das Auge eines Patienten eingebrachte Stützmembran 1 gibt es im Wesentlichen zwei Varianten. Gemäß einer Variante verbleibt die Stützmembran 1 nach Abschluss der Operation in der Vorderkammer 15 des Auges, bis sie vollständig im Körper abgebaut wird bzw. sich auflöst. Alternativ kann die Stützmembran 1, nachdem die Descemet-Membran 10 an die Hornhautrückwand 8 angewachsen ist, dem Auge wieder entnommen werden.

### Bezugszeichenliste

- 1: Stützmembran
- 2: Implantat
- 3: erste äußere Schicht
- 4: zweite äußere Schicht
- 5: mittlere Schicht
- 6: Substrat
- 7: Augenhornhaut
- 8: Rückwand der Augenhornhaut
- 9: Rand
- 10: Descemet-Membran
- 11: Endothelschicht
- 12: Verankerungszahn
- 13: Faser
- 14: Pore
- 15: Vorderkammer des Auges

## Patentansprüche

1. Stützmembran (1) für ein Implantat (2) zur Behandlung von Endothelerkrankungen einer Augenhornhaut (7) eines Menschen oder eines Tieres mit einem Substrat (6), das für eine Befestigung an einer Rückwand (8) der Augenhornhaut (7) geeignet ist,
**dadurch gekennzeichnet, dass** das Substrat (6) drei übereinander angeordnete Schichten (3, 4, 5) aufweist, von denen eine mittlere Schicht (5) in Form einer Folie ausgebildet ist und über einen Rand (9) verfügt, der zur Verankerung in der Augenhornhaut (7) geeignet ist, und dass beidseitig der mittleren Schicht (5) wenigstens bereichsweise auf dieser Schicht angeordnete äußere Schichten (3, 4) jeweils eine poröse, flüssigkeitsdurchlässige Faserstruktur aufweisen.

2. Stützmembran nach Anspruch 1,
**dadurch gekennzeichnet, dass** die mittlere und/oder wenigstens eine der äußeren Schichten (3, 4, 5) ein biokompatibles und/oder im Körper des Menschen oder Tieres auflösbares oder abbaubares Polymer aufweist.

3. Stützmembran nach Anspruch 1 oder 2,
**dadurch gekennzeichnet, dass** die mittlere Schicht (5) hydrophob und wenigstens eine der äußeren Schichten (3, 4) hydrophil ist.

4. Stützmembran nach einem der vorangehenden Ansprüche,
**dadurch gekennzeichnet, dass** wenigstens eine der äußeren Schichten (3, 4) ein gerichtetes Fasergewirk aufweist.

5. Stützmembran nach einem der vorangehenden Ansprüche,
**dadurch gekennzeichnet, dass** die mittlere und/oder wenigstens eine der äußeren Schichten (3, 4, 5) wenigstens ein Polyester, Polycaprolacton, Polylactid, Polysaccharid, Hyaluron-Derivat und/oder zumindest ein Komposit, Blend und/oder Copolymer der vorgenannten Materialien aufweist.

6. Stützmembran nach einem der vorangehenden Ansprüche,
**dadurch gekennzeichnet, dass** die mittlere Schicht (5) wenigstens ein lipophiles oder amphophiles Additiv aufweist.

7. Stützmembran nach einem der vorangehenden Ansprüche,
**dadurch gekennzeichnet, dass** der Rand (9) der mittleren Schicht (5) Polyhexamethylenadipinsäureamid, Polyamid, Kohlenstofffasern, Gold, Tantal, Silber, Platin und/oder Tetraferroplatin aufweist.

8. Stützmembran nach einem der vorangehenden Ansprüche,
**dadurch gekennzeichnet, dass** die mittlere und/oder wenigstens eine der äußeren Schichten (3, 4, 5) wenigstens über ein Element aus einer Stoffgruppe, die Färbemittel, Wachstumsfaktoren, Antibiotika, Virostatika, Hormone und Immunsuppressiva enthält, verfügt.

9. Stützmembran nach einem der vorangehenden Ansprüche,
**dadurch gekennzeichnet, dass** die mittlere Schicht (5) transparent für sichtbares Licht ist.

10. Stützmembran nach einem der vorangehenden Ansprüche,
**dadurch gekennzeichnet, dass** wenigstens eine der äußeren Schichten (3, 4) ausgebildet ist, um ein Zellwachstum in und/oder an der jeweiligen Schicht (3, 4) zu ermöglichen oder zu begünstigen.

11. Stützmembran nach einem der vorangehenden Ansprüche,
**dadurch gekennzeichnet, dass** eine Dicke des Substrats (6) nicht größer als 100 µm ist.

12. Stützmembran nach einem der vorangehenden Ansprüche,
**dadurch gekennzeichnet, dass** auf wenigstens einer der äußeren Schichten (3, 4) wenigstens bereichsweise eine Descemet-Membran (10) und/oder Endothelzellen (11), die von einem Spender stammen und/oder dort kultiviert wurden, angeordnet sind.

13. Verfahren zur Herstellung einer Stützmembran für ein Implantat zur Behandlung von Endothelerkrankungen einer Augenhornhaut (7) eines Menschen oder eines Tieres mit einem Substrat (6) mit den Schritten:
- Erzeugen einer ersten äußeren porösen, wasserdurchlässigen Schicht (3) aus einem ersten Material im Wege eines Elektrospinnvorgangs und Aufbringen der ersten äußeren Schicht (3) auf eine gewölbte Formoberfläche,
- Erzeugen einer homogenen, folienartigen mittleren Schicht (5) auf der ersten äußeren Schicht (3) durch Aufbringen eines zweiten Materials auf die erste äußere Schicht (3) sowie
- Erzeugen einer zweiten äußeren porösen, wasserdurchlässigen Schicht (4) aus dem ersten Material im Wege eines Elektrospinnvorgangs und Aufbringen der zweiten äußeren Schicht (4) auf die mittlere Schicht (5).

14. Verfahren nach Anspruch 13,
**dadurch gekennzeichnet, dass** die erste äußere Schicht (3), die mittlere Schicht (5) und die zweite äußere Schicht im Wege eines Elektrospinnvorgangs hergestellt und die erste äußere Schicht während oder nach dem Elektrospinnvorgang auf die gewölbte Formoberfläche aufgebracht wird.

15. Verfahren nach Anspruch 13 oder 14,
**dadurch gekennzeichnet, dass** die mittlere Schicht (5) durch Aufsprühen des zweiten Materials auf die erste äußere Schicht (3) erzeugt wird.

## Claims

1. A supporting membrane (1) for an implant (2) for the treatment of endothelial diseases of a cornea (7) of a human or an animal, having a substrate (6) suitable for attachment to a rear wall (8) of the cornea (7),
**characterized in that** the substrate (6) has three layers (3, 4, 5) arranged one above the other, of which a middle layer (5) is designed in the form of a film and has an edge (9) suitable for anchoring in the cornea (7), and **in that** outer layers (3, 4) arranged on both sides of the middle layer (5), at least in some areas on this layer, each have a porous, liquid-permeable fiber structure.

2. The supporting membrane according to claim 1,
**characterized in that** the middle and/or at least one of the outer layers (3, 4, 5) comprises a biocompatible polymer and/or a polymer dissolvable or degradable in the human or animal body.

3. The supporting membrane according to claim 1 or 2,
**characterized in that** the middle layer (5) is hydrophobic and at least one of the outer layers (3, 4) is hydrophilic.

4. The supporting membrane according to any one of the preceding claims, **characterized in that** at least one of the outer layers (3, 4) comprises a knitted fabric made of oriented fibers.

5. The supporting membrane according to any one of the preceding claims, **characterized in that** the middle and/or at least one of the outer layers (3, 4, 5) comprises at least one polyester, polycaprolactone, polylactide, polysaccharide, hyaluronic acid derivative and/or at least one composite, blend and/or copolymer of the aforementioned materials.

6. The supporting membrane according to any one of the preceding claims, **characterized in that** the middle layer (5) comprises at least one lipophilic or amphophilic additive.

7. The supporting membrane according to any one of the preceding claims, **characterized in that** the edge (9) of the middle layer (5) comprises polyhexamethylene adipamide, polyamide, carbon fibers, gold, tantalum, silver, platinum and/or tetraferroplatinum.

8. The supporting membrane according to any one of the preceding claims, **characterized in that** the middle and/or at least one of the outer layers (3, 4, 5) comprises at least one element from a group of substances containing colorants, growth factors, antibiotics, antivirals, hormones and immunosuppressants.

9. The supporting membrane according to any one of the preceding claims, **characterized in that** the middle layer (5) is transparent for visible light.

10. The supporting membrane according to any one of the preceding claims, **characterized in that** at least one of the outer layers (3, 4) is designed to enable or promote cell growth in and/or on the respective layer (3, 4).

11. The supporting membrane according to any one of the preceding claims, **characterized in that** a thickness of the substrate (6) is no greater than 100 µm.

12. The supporting membrane according to any one of the preceding claims, **characterized in that** on at least one of the outer layers (3, 4), at least in some areas, a Descemet membrane (10) and/or endothelial cells (11) are arranged, which originate from a donor and/or were cultivated there.

13. A method for manufacturing a supporting membrane for an implant for the treatment of endothelial diseases of a cornea (7) of a human or an animal having a substrate (6), the method comprising the steps of:
- creating a first outer porous, water-permeable layer (3) of a first material by means of an electrospinning process and applying the first outer layer (3) onto a curved mold surface,
- creating a homogeneous, film-like middle layer (5) on the first outer layer (3) by applying a second material onto the first outer layer (3), as well as
- creating a second outer porous, water-permeable layer (4) of the first material by means of an electrospinning process and applying the second outer layer (4) onto the middle layer (5).

14. The method according to claim 13,
**characterized in that** the first outer layer (3), the middle layer (5) and the second outer layer are manufactured by means of an electrospinning process, and **in that** the first outer layer is applied onto the curved mold surface during or after the electrospinning process.

15. The method according to claim 13 or 14,
**characterized in that** the middle layer (5) is created by spraying the second material onto the first outer layer (3).

## Revendications

1. Membrane de support (1) pour un greffon (2) destiné au traitement de maladies endothéliales de la cornée (7) d'un être humain ou d'un animal, comprenant un substrat (6) adapté pour être fixé à une couche postérieure (8) de la cornée (7), **caractérisée en ce que** ledit substrat (6) comporte trois couches (3, 4, 5) dont une couche médiane (5) est réalisée sous forme de film et comporte un bord (9) adapté à l'ancrage dans la cornée (7), et **en ce que** des couches extérieures (2, 3) qui sont disposées de part et d'autre de ladite couche médiane (5) (3, 4) et qui sont disposées au moins par zones sur cette couche, présentent chacune une structure fibreuse poreuse et perméable aux liquides.

2. Membrane de soutien selon la revendication 1,
**caractérisée en ce que** ladite couche médiane et/ou au moins l'une desdites couches extérieures (3, 4, 5) comprend un polymère biocompatible et/ou soluble ou dégradable dans le corps humain ou animal.

3. Membrane de support selon la revendication 1 ou 2,
**caractérisée en ce que** ladite couche médiane (5) est hydrophobe et au moins l'une desdites couches extérieures (3, 4) est hydrophile.

4. Membrane de support selon l'une des revendications précédentes, **caractérisée en ce qu'**au moins l'une desdites couches extérieures (3, 4) présente des fibres orientées formant une maille.

5. Membrane de support selon l'une des revendications précédentes, **caractérisée en ce que** ladite couche médiane et/ou au moins l'une desdites couches extérieures (3, 4, 5) comprend au moins un polyester, une polycaprolactone, un polylactide, un polysaccharide, un dérivé d'acide hyaluronique et/ou au moins un composite, un mélange et/ou un copolymère des matériaux précités.

6. Membrane de support selon l'une des revendications précédentes, **caractérisée en ce que** ladite couche médiane (5) comprend au moins un additif lipophile ou amphophile.

7. Membrane de support selon l'une des revendications précédentes,
**caractérisée en ce que** le bord (9) de ladite couche médiane (5) comprend du poly hexaméthylène adipamide, du polyamide, des fibres de carbone, de l'or, du tantale, de l'argent, du platine et/ou du tétraferroplatine.

8. Membrane de support selon l'une des revendications précédentes,
**caractérisée en ce que** ladite couche médiane et/ou au moins l'une desdites couches extérieures (3, 4, 5) comporte au moins un élément appartenant à un groupe de substances qui contient des colorants, des facteurs de croissance, des antibiotiques, des virostatiques, des hormones et des immunosuppresseurs.

9. Membrane de support selon l'une des revendications précédentes,
**caractérisée en ce que** ladite couche médiane (5) est transparente à la lumière visible.

10. Membrane de support selon l'une des revendications précédentes,
**caractérisée en ce qu'**au moins l'une desdites couches extérieures (3, 4) est conçue pour permettre ou favoriser la croissance cellulaire dans et/ou sur la couche respective (3, 4).

11. Membrane de support selon l'une des revendications précédentes,
**caractérisée en ce que** l'épaisseur du substrat (6) n'est pas supérieure à 100 µm.

12. Membrane de support selon l'une des revendications précédentes,
**caractérisée en ce qu'**au moins une membrane de Descemet (10) et/ou des cellules endothéliales (11) provenant d'un donneur et/ou cultivées à cet endroit sont disposées au moins par zones sur au moins l'une desdites couches extérieures (3, 4).

13. Procédé de fabrication d'une membrane de support pour un greffon destiné au traitement des maladies endothéliales de la cornée (7) d'un être humain ou d'un animal, comprenant un substrat (6) et composé des étapes suivantes :
- la création d'une première couche extérieure poreuse et perméable à l'eau (3) à partir d'un premier matériau au moyen d'un procédé d'électrofilage et l'application de ladite première couche extérieure (3) sur une surface de moulage bombée,
- la création d'une couche médiane homogène en forme de film (5) sur ladite première couche extérieure (3) par l'application d'un deuxième matériau sur ladite première couche extérieure (3), ainsi que
- la création d'une deuxième couche extérieure poreuse et perméable à l'eau (4) à partir du premier matériau au moyen d'un procédé d'électrofilage et l'application de ladite deuxième couche extérieure (4) sur ladite couche médiane (5).

14. Procédé selon la revendication 13,
**caractérisé en ce que** ladite première couche extérieure (3), ladite couche médiane (5) et ladite deuxième couche extérieure sont fabriquées au moyen d'un procédé d'électrofilage et ladite première couche extérieure est appliquée sur ladite surface de moulage bombée pendant ou après ledit procédé d'électrofilage.

15. Procédé selon la revendication 13 ou 14,
**caractérisé en ce que** ladite couche médiane (5) est créée par pulvérisation du deuxième matériau sur ladite première couche extérieure (3).
